# EUROPÄISCHE PATENTSCHRIFT

(11) **EP 1 003 887 B1**
(45) Veröffentlichungstag und Bekanntmachung des Hinweises auf die Patenterteilung: **13.11.2002**
(21) Anmeldenummer: 98941325.7
(22) Anmeldetag: 13.07.1998
(51) Int. Cl.: C12N 15/70, C12N 15/80, C12N 15/81, C12N 1/21, C12N 1/19

(54) **VERWENDUNG EINER VIRUS-DNA ALS PROMOTOR**
USE OF A VIRUS DNA AS PROMOTER
UTILISATION D'UN VIRUS ADN COMME PROMOTEUR

(30) Priorität: 16.07.1997 DE 19730502
(43) Veröffentlichungstag der Anmeldung: 31.05.2000
(73) Patentinhaber: Max-Planck-Gesellschaft zur Förderung der Wissenschaften e.V., 80539 München (DE)
(72) Erfinder: ROHDE, Wolfgang, D-80933 Köln (DE); BECKER, Dieter, D-50827 Köln (DE); RANDLES, John, W., Stirling, S.A. 5152 (AU); HEHN, Alain, F-54500 Vandocuvre (FR); SALAMINI, Francesco, D-50829 Köln (DE)
(74) Vertreter: Wibbelmann, Jobst, Dr., Dipl.-Chem.
(86) Internationale Anmeldenummer: EP9804345
(87) Internationale Veröffentlichungsnummer: WO99004020

(56) Entgegenhaltungen:
- WO-A-94/19472
- DE-C- 4 306 832
- US-A- 5 563 328
- HEHN, A., ET AL.: "Characterization of cis-acting elements affecting strength and phloem specificity of the coconut foliar decay virus promoter" JOURNAL OF GENERAL VIROLOGY, Bd. 79, Juni 1998, Seiten 1495-1499, XP002086138
- ROHDE, W., ET AL.: "The promoter of coconut foliar decay-associated circular single-stranded DNA directs phloem-specific reporter gene expression in transgenic tobacco" PLANT MOLECULAR BIOLOGY, Bd. 27, 1995, Seiten 623-628, XP002084820 in der Anmeldung erwähnt
- POBJECKY, N., ET AL.: "Expression of the beta-glucuronidase gene under the control of the CaMV 35S promoter in Schizosaccharomyces pombe" MOL. GEN. GENET., Bd. 220, 1990, Seiten 314-316, XP002084821 in der Anmeldung erwähnt
- ASSAAD, F.F., ET AL.: "Cauliflower mosaic virus P35S promoter activity in Escherichia coli" MOL. GEN. GENET., Bd. 223, 1990, Seiten 517-520, XP002084822 in der Anmeldung erwähnt
- DATABASE WPI Section Ch, Week 8833 Derwent Publications Ltd., London, GB; Class B04, AN 88-231509 XP002084911 & JP 63 164888 A (SUGIYAMA SANGYO KAGAKU KENKYUSHO), 8. Juli 1988
- DATABASE WPI Section Ch, Week 8652 Derwent Publications Ltd., London, GB; Class B04, AN 86-343148 XP002084912 & JP 61 257185 A (MITSUBISHI CHEM IND LTD) , 14. November 1986
- MITRA, A., ET AL.: "A Chlorella virus gene promoter functions as a strong promoter both in plants and bacteria" BIOCHEMICAL AND BIOPHYSICAL RESEARCH COMMUNICATIONS, Bd. 204, Nr. 1, 1994, Seiten 187-194, XP002084910 in der Anmeldung erwähnt
- MOROZOV, S. YU. ET AL: "Computer search of transcription control sequences in small plant virus DNA reveals a sequence highly homologous to the enhancer element of histone promoters" DNA SEQUENCE (1994), 4(6), 395-7 , XP002084929
- CHEMICAL ABSTRACTS, vol. 106, no. 3, 19. Januar 1987 Columbus, Ohio, US; abstract no. 13843, DEUSCHLE, ULRICH ET AL: "Promoters of Escherichia coli: a hierarchy of in vivo strength indicates alternate structures" XP002086139 & EMBO J. (1986), 5(11), 2987-94 ,

## Beschreibung

Es ist allgemein bekannt, daß sich mittels gentechnologischer Arbeitstechniken gezielt einzelne Gene in das Genom von Lebewesen, wie Mikroorganismen, Hefen oder Pflanzen, übertragen lassen. Diese als Transformation oder bei höheren Zellen auch als Transfection bekannte Technik wird routinemäßig auf verschiedenen Wegen durchgeführt, z.B. durch "particie gun bombardment" (vgl. M.E. Fromm, F. Morrish, C. Armstrong, R. Williams, J. Thomas und T.M. Klein: "Inheritance and expression of chimeric genes in the progeny of transgenic maize plants", Bio/Technology 8: 833-839, 1990), "nackten DNA-Transfer" (vgl. P. Meyer, I. Heidmann, G. Forkmann und H. Saedler: "A new petunia flower colour generated by transformation of a mutant with a maize gene", Nature 330: 677-678, 1987) oder durch Agrobacterium-vermittelte stabile Integration von Genen oder Genabschnitten in das Genom einer Empfängerpflanze. Als Alternative zur chromosomalen Integration von Fremdgenen können z.B. extrachromosomal replizierende Vektoren benutzt werden, um Fremdgene ohne Integration in einem gewünschten Organismus zu exprimieren. Für Pflanzen stehen hierfür beispielsweise extrachromosomal replizierende Vektoren zur Verfügung, die aus Pflanzenviren entwickelt wurden (vgl. z.B. J. W. Davies und J. Stanley: "Geminivirus genes and vectors", Trends Genet. 5: 77-81, 1989). Die in den gewählten Organismen zu exprimierenden Fremdgene müssen zu diesem Zweck unter die Kontrolle von für diesen Organismus geeigneten Regulationssignalen (Promotor, Terminator) gebracht werden, die für eine konstitutive oder induzierbare und/oder gegebenenfalls gewebe- und/oder entwicklungsspezifische Transkription sorgen. Außerdem ist es wünschenswert, durch Verwendung eines starken Promotors eine erhöhte mRNA-Synthese des Fremdgens zu bewirken.

Die Regulationssignale für die Transkription von zu exprimierenden Genen, die in Bakterien und Eukaryoten gefunden werden, weisen bekanntermaßen große Unterschiede auf (vgl. z.B. Mitra et al., 1994, Biochem. Biophys. Res. Commun. 204: 187-194; Pobjecky et al., 1990, Mol. Gen. Genet. 220: 314-316). Hinsichtlich Promotoren enthalten zwar auch Eukaryoten eine der -10-Sequenz der Prokaryoten (TATAAT) entsprechende, sehr ähnliche TATA- oder Hogness-Box, jedoch ist diese bei Eukaryoten weiter vom Transkriptionsstartpunkt entfernt. Hinzu kommen bei den Eukaryoten zusätzliche Elemente, die für die Promotoraktivität unerläßlich sind. Aus diesem Grunde sind aus Eukaryoten stammende Promotoren in der Regel zur Regulation der Transkription von Genen in Prokaryoten nicht geeignet und umgekehrt. Auch innerhalb der Eukaryoten gibt es Unterschiede bei den Promotorstrukturen, z.B. zwischen Pflanzen und Pilzen, sowie auch z.B. hinsichtlich der Spezifität eines Promotors für bestimmte Zellen innerhalb ein und desselben eukaryotischen Organismus.

Es wäre für Wirtschaft und Forschung überaus nützlich, über Promotoren zu verfügen, die in Prokaryoten wie Eukaryoten, also beispielsweise in Bakterien, Pilzen und Pflanzen, gleichermaßen aktiv sind. Dies würde beispielsweise aufwendige Umklonierungsarbeiten bei beabsichtigtem gleichzeitigen Einsatz von prokaryotischen und eukaroytischen Empfängerorganismen für die Expression von Fremdgenen ersparen.

Ein derartiger Promotor ist der 35S-RNA-Promotor des cauliflower mosaic virus (CaMV). Dieser Promotor erfüllt die Anforderung an einen starken, konstitutiven Promotor in Pflanzenzellen und wird vorwiegend für die Pflanzentransformation eingesetzt (vgl. R. Walden: "Genetic Transformation in Plants", Open University Press, Milton Keynes, 1988). Es ist jedoch bekannt, daß der CaMV 35S-Promotor auch in Bakterien aktiv ist (Assaad und Signer, Molecular and General Genetics 223: 517-520, 1990).

Es ist dementsprechend Aufgabe der Erfindung, weitere von einem pflanzenspezifischen Virus abgeleitete Promotoren bereitzustellen, die in Bakterien und/oder Pilzen aktiv sind. Vorteilhafterweise sind diese zugleich in Pflanzen und insbesondere gleichermaßen in Pflanzen und/oder Pilzen und Bakterien aktiv, so daß sie sowohl in Eukaryoten als auch in Prokaryoten eingesetzt werden können. Vorteilhafterweise weisen sie darüberhinaus zusätzlich eine gegenüber dem CaMV 355-Promotor stärkere Promotoraktivität in Pflanzen, Pilzen und/oder Bakterien auf.

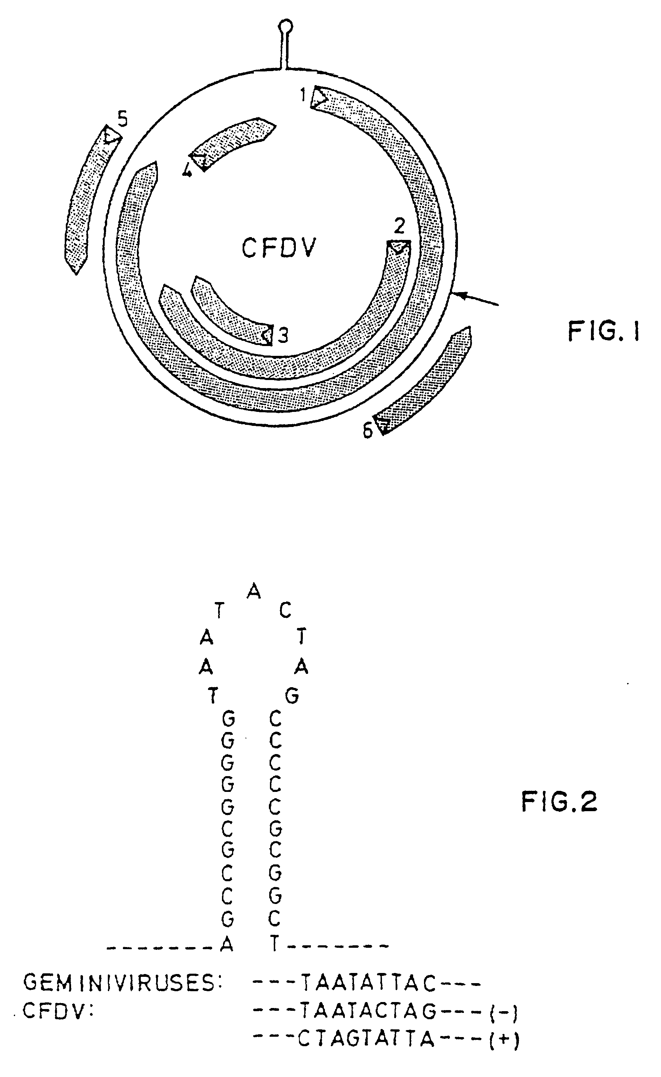

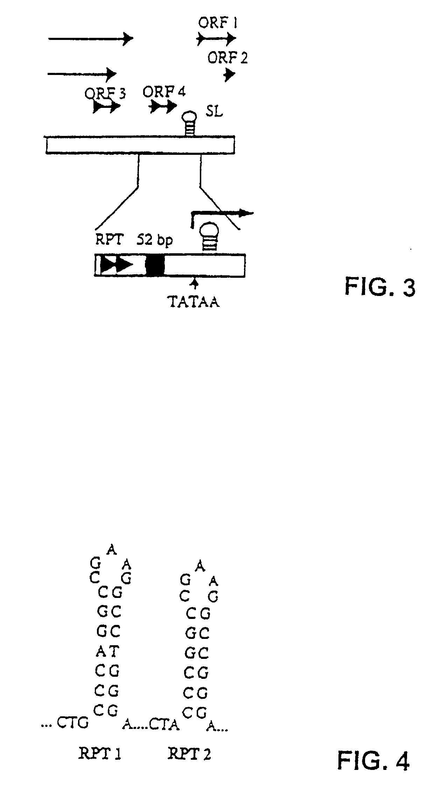

In dem deutschen Patent DE 43 06 832 der Max-Planck-Gesellschaft zur Förderung der Wissenschaften sowie in Rohde et al., Plant Molecular Biology 27: 623-628, 1995, wurde die Verwendung einer aus dem die Kokospalme Cocos nucifera befallenden Virus CFDV (coconut foliar decay virus) stammenden DNA, deren Struktur in den Figuren 1, 3A und 3B der Patentschrift dargestellt ist, als viraler phloemspezifischer Promotor zur gewebespezifischen Expression von Genen in transgenen Pflanzen beschrieben.

Das CFDV-Virus ist im vaskulären System der Pflanze lokalisiert (vgl. J.W. Randles et al.: " Localization of coconut foliar decay virus in coconut palm", Ann. Appl. Biology 1992, 601-617). Eine mit den Krankheitssymptomen und dem Auftreten von Viruspartikeln assoziierte DNA war bereits früher kloniert, sequenziert und ihre Struktur bestimmt worden (vgl. W. Rohde et al.: "Nucleotide sequence of a circular single-stranded DNA associated with coconut foliar decay virus", Virology 176: 648-651, 1990). CFDV ist ein virales Phytopathogen mit einem Genom aus einzelsträngiger, zirkulärer, kovalent geschlossener DNA. In Rohde et al., Virology 176: 648-651, 1990, wurden ein DNA-Molekül des CFDV mit einer Größe von 1291 Nukleotiden sowie Deletionsmutanten davon beschrieben. CFDV ist kein Vertreter der Geminivirus-Gruppe, sondern bildet vermutlich den Prototyp der DNA-Virusgruppe der "Circoviren".

Überraschenderweise wurde nun herausgefunden, daß die CFDV-DNA und Fragmente der CFDV-DNA auch in Bakterien und Pilzen als Promotoren aktiv sind. So ist die Promotoraktivität beispielsweise in E. coli deutlich höher als die des gleichfalls in Bakterien aktiven CaMV 35S-Promotor (Assaad und Signer, Molecular and General Genetics 223: 517-520, 1990); die CFDV-Konstrukte pRT CF4 und pRT CF9 weisen in E. coli sogar eine bis zu 60-fach höhere Aktivität als der CaMV 35S-Promotor auf; auch in Tabak-Protoplasten zeigt der in dem Konstrukt pRT CF4 enthaltene CDFV-Fragment-Promotor eine geringfügig höhere Promotoraktivität als der CaMV 35S-Promotors. Aufgrund dieser Aktivität eignen sich die CFDV-Promotoren insbesondere für den Einsatz in bakteriellen Systemen und Pilzsystemen, beispielsweise zur Herstellung von pharmakologisch aktiven Proteinen oder Peptiden.

Gegenstand der Erfindung ist demzufolge die in den Ansprüchen gekennzeichnete Verwendung der CFDV-DNA und von CFDV-DNA-Fragmenten als bakterielle Promotoren sowie als Promotoren in Pilzen, insbesondere in Hefen.

Zur Herstellung der für die erfindungsgemäße Verwendung geeigneten CFDV-DNA-Fragmente bedient man sich dem Fachmann wohlbekannter Techniken, wie beispielsweise geeigneter Schnittstellen von Restriktionsendonukleasen auf der CFDV-DNA oder der Polymerase-Kettenreaktionstechnik, die es ermöglicht, mittels spezifischer Primer CFDV-DNA-Fragmente der gewünschten Länge ausgehend von einem Vollängen-CFDV-DNA-Konstrukt zu amplifizieren. Hierzu werden auf an sich bekannte Weise die Primer entsprechend dem gewünschten CFDV-Fragment anhand der von W. Rohde et al. in Virology 176: 648-651, 1990 beschriebenen Nukleotidsequenz des CFDV-Virus und genauer der Nukleotidsequenzen im Bereich der 5'- bzw. 3'-Enden des gewünschten Fragments synthetisiert.

Für die erfindungsgemäße Verwendung bevorzugte CFDV-DNA-Fragmente sind die DNA-Fragmente mit den Nukleotiden 211 bis 991, 409 bis 991, 611 bis 991, 711 bis 991, 211 bis 962, 409 bis 962, 611 bis 962 und 711 bis 962 sowie das *Xho*I/*Sty*I-Fragment der CFDV-DNA mit den Nukleotiden 1 bis 1157.

Für die erfindungsgemäße Verwendung ganz besonders bevorzugte CFDV-DNA-Fragmente sind DNA-Fragmente, die lediglich den Sequenzabschnitt der CFDV-DNA einschließlich der wiederholten Sequenz (RPT), der 52 bp-Sequenz und der TATAA-Box umfassen, ohne den Sequenzabschnitt bis zum Ende des offenen Leserasters ORF1 und sämtliche für den Aufbau der sogenannten "stem-loop"-Struktur erforderlichen Nukleotide zu umfassen. Dementsprechend ganz besonders bevorzugte DNA-Fragmente sind die DNA-Fragmente mit den Nukleotiden 611 bis 991 und 611 bis 962.

Gleichfalls Gegenstand der Erfindung ist die erfindungsgemäße Verwendung von CFDV-DNA-Derivaten oder CFDV-Fragment-Derivaten, die von der CFDV-DNA oder von CFDV-Fragmenten durch Substitution, Deletion, Insertion oder Modifizierung von einzelnen Nukleotiden oder kleineren Gruppen von Nukleotiden abgeleitet sind und die eine vergleichbare Promotoraktivität wie die CFDV-DNA oder die Ausgangsfragmente aufweisen. Als vergleichbare Promotoraktivität kann beispielsweise eine Promotoraktivität angesehen werden, die bis zu 20% über oder unter derjenigen der CFDV-DNA oder des Ausgangsfragments liegt.

Die Figuren zeigen in:
- Fig. 1:: die schematische Struktur der CFDV-DNA mit 6 möglichen offenen Leserastern (ORF1-6) und der sogenannten "stem-loop"-Struktur. Der Pfeil weist auf die *Xho*I-Schnittstelle hin.
- Fig. 2:: die sogenannte "stem-loop"-Struktur; sie zeigt Homologie zu einer ähnlichen Struktur im Genom von Geminiviren und ist vermutlich für die Replikation des Virus verantwortlich.
- Fig. 3:: die schematische Anordnung möglicher Signale für die Transkriptions-Regulation auf der durch Schnitt an der *Xho*I-Schnittstelle linearisierten CFDV-DNA. Die Pfeile kennzeichnen die größeren offenen Leseraster ORF1, ORF2, ORF3 und ORF4 auf der CFDV-DNA. Die mit TATAA gekennzeichnete Stelle umfaßt eine mögliche TATA-Box, die zwei Pfeilköpfen zugeordnete Abkürzung RPT weist auf eine wiederholte Sequenz hin; die "stem-loop"-Struktur ist mit SL gekennzeichnet.
- Fig. 4:: die Sequenz der zwei wiederholten Sequenzen (RPT) und deren Anordnung als stabile "stem-loop"-Strukturen mit der üblichen CGAAG-loop-Sequenz.

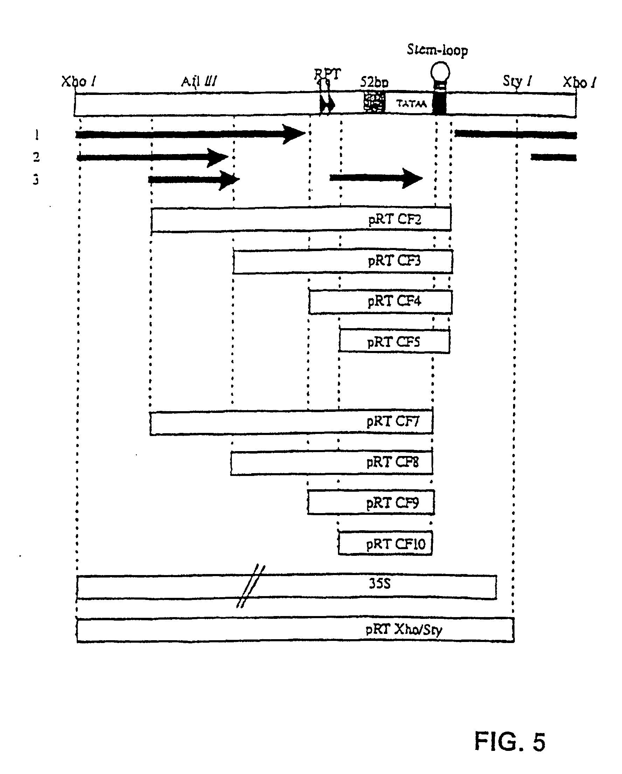

- Fig. 5:: eine schematische Darstellung der Lage von für Konstrukte zur Bestimmung der Promotorstärke verwendeten verschiedenen CFDV-Fragmenten auf der durch Schnitt an der *Xho*I-Schnittstelle linearisierten CFDV-DNA. Die Pfeilköpfe zeigen die Lage der zwei direkt wiederholten Sequenzen (RPT) oberhalb eines 52 bp-Elements (schwarzer Kasten) an. Dieses Element zeigt 70% Sequenzidentität zwischen CoYMV und CFDV. Die Pfeile kennzeichnen größere offene Leseraster in den drei Leserastern 1, 2 und 3 (ORF1, ORF2, ORF3) der CFDV-DNA. Die Abkürzung TATAA weist auf eine mögliche TATA-Box hin, die Lage der "stem-loop"-Struktur ist gleichfalls angegeben. *Xho*I, *Afl*III und *Sty*I kennzeichnen die Lage von Schnittstellen für Restriktionsendonukleasen.
- Fig. 6:: die schematische Struktur des Ausgangsplasmids pRTsynLUC.

### Untersuchungen zur Promotorstärke verschiedener CFDV-Fragmente in Pflanzen und Bakterien

Für Untersuchungen zur Promotorregion und -stärke durch die transiente Expression in Pflanzenzellen und Bakterien wurden Fragmente der CFDV-DNA, für die nicht auf geeignete Restriktionsschnittstellen auf der CFDV-DNA zurückgegriffen werden konnte, ausgehend von einem CFDV-Konstrukt voller Länge (Rohde et al., Plant Mol. Biol. 27: 623-628, 1995) zunächst mittels der Polymerasekettenreaktion (PCR) amplifiziert und als subgenomische Fragmente in dem Plasmidvektor pRT2synGUSΔH in transkriptionelle Fusion zum Gen der β-Glucuronidase (GUS) gebracht.

Die das Gen der β-Glucuronidase in Fusion mit der CFDV-Gesamtsequenz oder dem *Xho*I/*Sty*I-Fragment der CFDV-DNA enthaltenden Konstrukte wurden wie in DE 43 06 832 beschrieben hergestellt. Die erhaltenen Plasmide wurden in Experimenten zur transienten Expression mit einem entsprechenden CaMV 35S-Konstrukt verglichen.

### Anwendungsbeispiel

Sämtliche nachfolgend aufgeführten Verfahrenschritte wurden, sofern nicht anders angegeben, entsprechend Standardverfahren, wie sie beispielsweise in Sambrook et al., Molecular Cloning, A Laboratory Manual, Cold Spring Harbor Laboratory, Cold Spring Harbor, New York, USA (1989) beschrieben werden, ausgeführt.

### I. Herstellung von CFDV-(Fragment-)GUS-Konstrukten für die transiente Expression

Für Konstrukte mit CFDV-Fragmenten, für die nicht auf geeignete Restriktionsschnittstellen auf der CFDV-DNA zurückgegriffen werden konnte, diente als Ausgangsplasmid das CFDV-Konstrukt voller Länge, das in Rohde et al., Plant Mol. Biol. 27: 623-628, 1995 beschrieben wurde. Mit Hilfe spezifischer Primer, die zusätzliche Restriktionsschnittstellen enthielten, und zwar *Hind*III für das 5'-Ende und *Nco*I für das 3'-Ende der amplifizierten DNA-Moleküle, wurde das CFDV-Genom amplifiziert. Entsprechend der Wahl der Primer wurden hinsichtlich ihrer Länge festgelegte CFDV-Fragmente erhalten. Die Primer wurden anhand der von W. Rohde et al. in Virology 176: 648-651, 1990 beschriebenen Nukleotidsequenz des CFDV-Virus und zwar genauer anhand der Nukleotidsequenzen im Bereich der 5'- bzw. 3'-Enden des gewünschten Fragments synthetisiert, um durch die spätere DNA-Amplifikation die in der nachfolgenden Tabelle 1 aufgeführten CFDV-Fragmente zu gewinnen. Die Primer waren zusätzlich um DNA-Abschnitte ergänzt, die die vorstehend genannten zusätzlichen Restriktionsschnittstellen enthielten.

Die Amplifikationsprodukte wurden mit *Hind*III/*Nco*I verdaut, die Spaltungsprodukte in einem Agarosegel aufgetrennt und die gewünschten DNA-Fragmente durch Elektroelution isoliert.

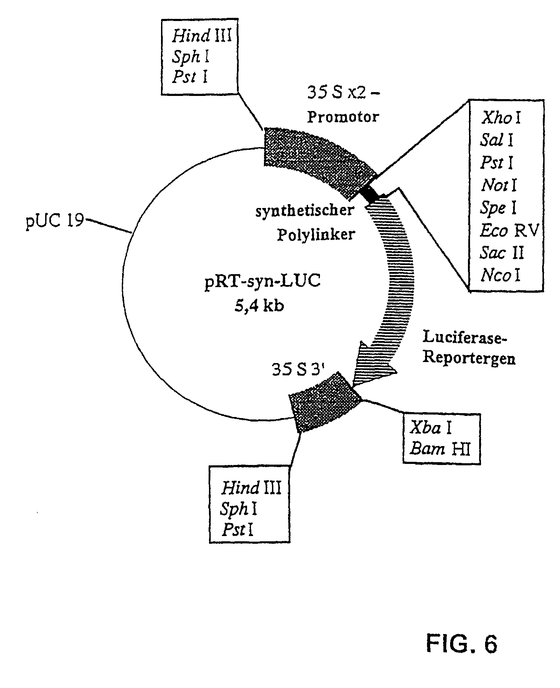

Die CFDV-Fragmente wurden dann in den Vektor pRT2synGUSΔH ligiert, der vorab aus dem,Plasmid pRTsynLUC (Fig. 6; Turner et al., Arch. Virol. 137: 123-132, 1994) hergestellt worden war. Dazu wurde das Luziferase-Gen durch *Nco*I/*Bam*HI-Verdau entfernt und durch das GUS-Gen mit *Nco*I/*Bam*HI-Enden ersetzt. Schließlich wurde die HindIII-Schnittstelle am 35S 3'-Ende deletiert, indem das Plasmid partiell mit *Hind*III geschnitten, die Schnittstelle aufgefüllt und das lineare Molekül durch Religation zu pRT2synGUSΔH zirkularisiert wurde. Anstelle der *Hind*III-Schnittstelle wurde so eine *Nhe*I-Schnittstelle geschaffen. Der 35S-Promotor wurde aus diesem Plasmid durch *Hind*III/*Nco*I-Verdau entfernt und durch die entsprechenden *Hind*III/*Nco*I-CFDV-Fragmente ersetzt.

Die das Gen der β-Glucuronidase in Fusion mit der CFDV-Gesamtsequenz oder dem *Xho*I/*Sty*I-Fragment der CFDV-DNA enthaltenden Konstrukte wurden, wie in DE 43 06 832, Kapitel "Untersuchungen zur Promotorregion und -stärke" sowie Kapitel I.2 des Anwendungsbeispiels beschrieben, hergestellt. Hierzu wurde die 1291 Nukleotide große CFDV-cDNA (vgl. Rohde et al., Virology 176: 648-651, 1990) aus einem Plasmid mit dem Restriktionsenzym *Xho*I herausgeschnitten und als Gesamtsequenz oder nach zusätzlichem Verdau mit der Restriktionsendonuklease *Sty*I nach Auftrennung im Agarosegel und Elektroelution als subgenomische Fragmente im Plasmidvektor pUC19GUS in transkriptionelle Fusion zum Gen der β-Glucuronidase gebracht. Zur Konstruktion von pUC19GUS wurde von dem Vektor pUC103GUS ausgegangen, der aus dem Vektor pRT103GUS (Töpfer, R., Pröls, M., Schell, J. und Steinbiss, H.H., Plant Cell Rep., 1988, 7: 225-228) durch PstI-Verdau und Subklonierung des GUS-Fragments in pUC19(*Pst*I) hergestellt wurde. Aus pUC103GUS wurde durch Verdau mit den Restriktionsenzymen *Hind*III und *Xho*I der CaMV35S-Promotor entfernt und dessen Enden in "flush"-Enden überführt. Daraus wurden durch kovalentes Verknüpfen mit der vorstehend erhaltenen linearisierten CFDV-Gesamtsequenz bzw. dem *Sty*I-Fragment mittels T4-induzierter DNA-Ligase die entsprechenden Konstrukte hergestellt.

Die in den erzeugten CFDV-Fragment-GUS-Konstrukten als Promotoren enthaltenen CFDV-Fragmente sind hinsichtlich ihrer exakten Lage auf der CFDV-DNA in Tabelle 1 aufgeführt und schematisch in Figur 4 gezeigt. Die in Tabelle 1 angegebenen Nukleotidpositionen beziehen sich auf eine durch Spaltung mit der Restriktionsendonuklease *Xho*I linearisierte CFDV-DNA, deren 5'-Ende die Position 1 zugewiesen wurde. Zur Ergänzung wurden auch die entsprechenden DNA-Abschnitte für die "stem-loop"-Struktur, die offenen Leseraster ORF1 und ORF2 sowie weitere Strukturelemente der CFDV-DNA aufgenommen.

Die in Tabelle 1 enthaltenen und in Figur 4 schematisch dargestellten, mit pRT CF2 - 5 bezeichneten CFDV-Fragmente umfassen sämtlich die 52 bp-Sequenz, die TATAA-Box und die sogenannte "stem-loop"-Struktur der CFDV-DNA. Bei den mit pRT CF7-10 bezeichneten CFDV-Fragmenten handelt es sich um Fragmente von CFDV, die zwar sämtlich noch die 52 bp-Sequenz und die TATAA-Box umfassen, bei denen jedoch die CFDV-Sequenz an ihrem 3'-Ende derart deletiert ist, daß eine Ausbildung der "stem-loop"-Struktur nicht mehr möglich ist. Die mit pRT CF4 und 9 bezeichneten CFDV-Fragmente umfassen beide zusätzlich die RPT-Sequenzen, die mit pRT CF2, 3, 7 und 8 bezeichnete CFDV-Fragmente erstrecken sich noch weiter in 5'-Richtung.

**TABELLE 1**

| KONSTRUKT | 5'-Ende des CFDV-Fragments | 3'-Ende des CFDV-Fragments |
|---|---|---|
| pRT CF2 | 211 | 991 |
| pRT CF3 | 409 | 991 |
| pRT CF4 | 611 | 991 |
| pRT CF5 | 711 | 991 |
| pRT CF7 | 211 | 962 |
| pRT CF8 | 409 | 962 |
| pRT CF9 | 611 | 962 |
| pRT CF10 | 711 | 962 |
| pRT XhoI/StyI | 1 | 1157 |
| | | |
| RPT1 | 655 | 676 |
| RPT2 | 682 | 701 |
| 52 bp-Sequenz | 734 | 785 |
| TATA-Box | 934 | 939 |
| SL | 941 | 971 |
| ORF1 | 1004 | 583 |
| ORF2 | 1215 | 383 |

Das zu Vergleichszwecken eingesetzte Konstrukt pRT 35S, das das GUS-Reportergen in Fusion mit dem CaMV 35S-Promotor enthält, wurde, wie in dem deutschen patent P 43 06 832 beschrieben, als Vektor pUC103GUS aus dem Vektor pRT103GUS (Töpfer, R., Pröls, M., Schell, J. und Steinbiss, H.H., Plant Cell Rep., 1988, 7: 225-228) durch *Pst*I-Verdau und Subklonierung des GUS-Fragments in pUC19(*Pst*I) hergestellt.

### II. Transiente Expression von CFDV-Fragment-GUS-Konstrukten in E. coli

### II.1. Transformation von E. coli

Kompetente *E. coli* JM109-Zellen wurden mit den entsprechenden Plasmid-DNAs durch Elektroporation transformiert und auf LB-Platten (unter Zusatz von Ampicillin) selektioniert.

### III.2. Analyse der E. coli-Transformationen

Man ließ einzelne Kolonien in 2 ml LB-Medium (unter Zusatz von Ampicillin) über Nacht hochwachsen. Je 10 µl Bakteriensuspension wurden mit 35 µl Extraktionspuffer (50 mM Natriumphosphat-Puffer, pH 7; 10 mM EDTA, 0,1% Triton X-100) aufgeschlossen, mit 5 µl 10x 4-MUG-Lösung (4-MUG: 4-Methylumbelliferyl-β-Dglucuronid; vgl. R.A. Jefferson: "Assaying chimeric genes in plants: the GUS gene fusion system", Plant Mol. Biol. Rep. 5: 387-405, 1987) versetzt und bei 37°C 10 min oder für die Messung des Zeitverlaufs der GUS-Aktivität 10 min, 20 min oder 47 min bei 37°C inkubiert. Die Reaktion wurde durch Zugabe von 1 ml 0,2 M Na₂CO₃-Puffer gestoppt und die GUS-Aktivität mit 4-Methylumbelliferyl-β-D-glucuronid fluorimetrisch bestimmt. Die Proteinmenge wurde nach Bradford (vgl. M. Bradford: "A rapid and sensitive method for the quantitation of microgramme quantities of protein utilizing the principle of protein dye binding", Anal. Biochem. 72: 248-254, 1976) bestimmt.

Die für die einzelnen Konstrukte erhaltenen Ergebnisse sind in den folgenden Tabellen 2A und 2B aufgeführt. Die Ergeb nisse in Tabelle 2A sind angegeben als Prozentsatz an Aktivität bezogen auf die Aktivität des CFDV-Promotor-Konstrukts pRT CF4, die als die insgesamt höchste in diesem Beispiel erzielte Promotoraktivität zu 100% gesetzt wurde. Es werden die Ergebnisse von zwei oder drei unabhängigen Experimenten sowie der Mittelwert aus diesen Ergebnissen angegeben. Die in Tabelle 2B in der jeweils rechten Spalte für die einzelnen Inkubationsdauern angegebenen Prozentangaben geben den Prozentsatz an Aktivität bezogen auf diejenige des CFDV-Promotorkonstrukts pRT CF4 entsprechend den in den jeweils linken Spalten aufgeführten Absolutwerten für ausgewählte Konstrukte wieder.

**TABELLE 2A**

| KONSTRUKT | Exp. 1 | Exp. 2 | Exp. 3 | Mittelwert |
|---|---|---|---|---|
| pRT CF2 | 4,4 | 15,8 | 17,1 | 12,4 |
| pRT CF3 | 5,7 | 14,0 | 12,6 | 10,7 |
| pRT CF4 | 100 | 100 | 100 | 100 |
| pRT CF5 | 5,1 | 14,9 | - | 10,0 |
| pRT CF7 | 17,3 | 11,5 | 7,9 | 12,2 |
| pRT CF8 | 12,4 | 24,2 | 25,7 | 20,7 |
| pRT CF9 | 84,4 | 111,7 | 141,8 | 112,6 |
| pRT CF10 | 4,0 | 11,3 | 26,7 | 14,0 |
| pRT CF XS | 6,6 | 20,8 | 15,9 | 14,4 |
| pRT 35S | 3,6 | 11,3 | 8,6 | 7,8 |

**TABELLE 2B**

| KONSTRUKT | 10 min Inkubation | | 20 min Inkubation | | 47 min Inkubation | |
|---|---|---|---|---|---|---|
| pRT CF4 | 35.560 | 100 | 78.900 | 100 | 407.400 | 100 |
| pRT CF5 | 1.396 | 3,9 | 2.900 | 3,6 | 12.980 | 3,2 |
| pRT CF9 | 27.650 | 77,7 | 75.900 | 96,2 | 405.500 | 99,5 |
| pRT CF XS | 2.040 | 5,7 | 4.820 | 6,1 | 37.400 | 9,2 |
| pRT 35S | 1.222 | 3,4 | 1.766 | 2,2 | 6.820 | 1,7 |

Die in Tabelle 2A aufgeführten Ergebnisse zeigen, daß sämtliche genannten CFDV-DNA-Fragmente auch in Bakterien als Promotor aktiv sind und bei 10 minütiger Inkubation eine höhere Aktivität als der CaMV 35S-Promotor (vgl. Konstrukt pRT 35S) zeigen. Gegenüber dem Konstrukt pRT CF4, das als Promotor ein CFDV-DNA-Fragment enthält, das die wiederholte Sequenz (RPT), die 52 bp-Sequenz, die TATAA-Sequenz und die "stem-loop"-Struktur im Bereich der Nukleotide 941 bis 971 umfaßt, jedoch keinerlei DNA-Abschnitte der offenen Leseraster ORF1, ORF2 und auch ORF3 enthält, zeigt das Konstrukt pRT 35S mit dem CaMV 35S-Promotor nur weniger als 10% von dessen Aktivität. Das Konstrukt pRT CF9, das sich von dem Konstrukt pRT CF4 dadurch unterscheidet, daß es ein CFDV-Fragment umfaßt, dessen Sequenz an dem 3'-Ende derart deletiert ist, daß eine Ausbildung der "stem-loop"-Struktur nicht mehr möglich ist, zeigt eine vergleichbar hohe Aktivität. Die Ausbildung der "stem-loop"-Struktur scheint dementsprechend für die Promotoraktivität der aufgeführten Fragmente in Bakterien nicht erforderlich zu sein.

Bei fortgesetzter Inkubation (Tabelle 2B) nimmt die Aktivität der meisten in Tabelle 2B aufgeführten CFDV-Fragment-GUS-Konstrukte zu, wohingegen diejenige des CaMV 35S-Promotors konstant auf einem sehr niedrigen Wert bleibt. Am Ende der 47-minütigen Inkubationsdauer zeigt der CaMV 35S-Promotor lediglich nurmehr ungefähr 2 % der Aktivität der CFDV-Fragment-Promotoren der Konstrukte pRT CF4 und 9.

### III. Transiente Expression von CFDV-Fragment-GUS-Konstrukten in Tabak-Protoplasten

### III.1. Protoplastenmedien

| K3 | Makroelemente: | Mikroelemente: |
|---|---|---|
| | 25 mM KNO₃ | 100 µM H₃BO₃ |
| | 1 mM NaH₂PO₄ | 130 µM MnSO₄ |
| | 6 mM CaCl₂ | 40 µM ZnSO₄ |
| | 3 mM NH₄NO₃ | 5 µM KCl |
| | 1 mM (NH₄)₂SO₄ | 1 µM CuSO₄ |
| | 1 mM MgSO₄ | 1 µM COCl₂ |
| | | |

| | Eisen in EDTA: | Vitaminlösung: |
|---|---|---|
| | 1 µM FeSO₄ | 270 µM Glycin |
| | 1 µM Na₂EDTA | 160 µM Nicotinsäure |
| | | 100 µM Pyridoxin |
| | | 3 µM Thiamin |
| | | |

| | Kohlenhydrate: | Hormone: |
|---|---|---|
| | 400 mM Saccharose | 5,5 µM NAA |
| | 1,7 mM Xylose | 1,0 µM Kinetin |
| | 0,5 mM Inosit | |
| | | |
| | pH 5,6 osmotischer Wert: 600 mOs | |
| | | |
| W5 | 150 mM NaCl | |
| | 125 mM CaCl₂ | |
| | 5 mM KCl | |
| | 5 mM Glucose | |
| | | |
| | pH 5,6 - 6,0 | |
| | | |
| MaMg | 450 mM Mannitol | |
| | 15 mM MgCl₂ | |
| | 0,1 % MES | |
| | | |
| | pH 5,6 | |

### III.2. Herstellen von Tabak-Protoplasten (Vgl. I. Negrutiu et al., "Fusion of plant protoplasts: a study using auxotrophic mutants of Nicotiana plumbagenifolia, viviani", Theor. Appl. Genet. 72: 279-286, 1987).

Blätter (10 g) von in Gewebekultur herangezogenen Nicotiana tabacum-Pflanzen (var. SR1) wurden in 100 ml Enzymlösung für 16 h bei 25°C im Dunkeln inkubiert und die erhaltenen Protoplasten durch Siebe (Maschenweite 100 µm) von groben Geweberesten abgetrennt. Die weitere Aufreinigung der Protoplasten erfolgte durch wiederholte Zentrifugationen und Waschen mit K3-Medium, wobei sich die vitalen Protoplasten jeweils an der Oberfläche konzentrierten, sowie schließlich durch Resuspension in W5-Medium und Sedimentation durch Zentrifugation. Das Protoplasten-Sediment wurde in MaMg-Puffer aufgenommen und auf eine Konzentration von 10⁶/ml eingestellt.

### III.3. Transformation von Protoplasten (vgl. C. Maas und W. Werr: "Mechanism and optimized conditions for PEG mediated DNA-transfection into plant protoplasts", Plant Cell Rep. 8: 148-151, 1989).

Zu jeweils 500 µl Protoplasten wurden 15 µl Plasmid/Carrier-DNA (entsprechend 10 µg CFDV-Fragment-GUS-Konstrukt- bzw. CaMV35S-GUS-Plasmid-DNA und 50 µg Kalbsthymus-DNA) gegeben, die Suspension für 10 min bei Raumtemperatur inkubiert, danach vorsichtig mit PEG-Lösung (40% PEG 4000, 0,1 M Ca(NO₃)₂, 0,4 M Mannitol) unterschichtet und sofort geschwenkt, bis keine Schlieren mehr sichtbar waren. Nach weiterer 30-minütiger Inkubation erfolgte die Zugabe von 4 ml K3-Medium (mit Antibiotikum und Kinetinen) und die einzelnen Transformationsansätze wurden für 20 h bei 25°C im Dunkeln gehalten.

### III.4. Analyse der Protoplasten-Transformationen

Die Protoplasten-Ansätze wurden nach 20 h mit W5-Medium auf 10 ml aufgefüllt, zentrifugiert, die sedimentierten Protoplasten nach Resuspension in 1 ml W5-Medium erneut abzentrifugiert und in flüssigem Stickstoff eingefroren. Zur Bestimmung von Proteinmenge und GUS-Enzymaktivität mörserte man die Protoplasten in 50 µl GUS-Extraktionspuffer und bestimmte die GUS-Aktivität fluorimetrisch mit 4-Methylumbelliferyl-β-D-glucuronid (4-MUG; vgl. R.A. Jefferson, Plant Mol. Biol. Rep. 5: 387-405, 1987). Hierzu wurde mit 4-Methylumbelliferyl-β-Dglucuronid (4-MUG) 1 h bei 37°C inkubiert. Die Proteinmenge wurde nach Bradford bestimmt (vgl. M. Bradford, Anal. Biochem. 72: 248-254, 1976).

Die für die einzelnen Konstrukte erhaltenen Ergebnisse sind in der folgenden Tabelle 3 aufgeführt. Die Ergebnisse in Tabelle 3 sind angegeben als Prozentsatz an Aktivität der einzelnen CFDV-Konstrukte bezogen auf diejenige des CaMV 35S-Promotor-Konstrukts (pRT 35S), die zu 100% gesetzt wurde. Es werden die Ergebnisse von zwei oder drei unabhängigen Experimenten wie auch der Mittelwert aus diesen Ergebnissen angegeben. Das Konstrukt pRT CF XS enthält das in dem deutschen Patent P 43 06 832 erstmals offenbarte CFDV-Fragment, das durch die Spaltung der CFDV-DNA mittels der Restriktionsendonukleasen *Xho*I und *Sty*I erhalten wird und zusätzlich den Translationsstart des offenen Leserasters ORF1 mit umfaßt.

**TABELLE 3**

| Konstrukt | Exp. 1 | Exp. 2 | Exp. 3 | Mittelwert |
|---|---|---|---|---|
| pRT CF2 | - | 48 | 67 | 57,5 |
| pRT CF3 | 20 | - | 21 | 20,5 |
| pRT CF4 | 204 | 59 | 36 | 118 |
| pRT CF5 | 25 | 30 | 18,9 | 24,6 |
| pRT CF7 | 0 | 0 | - | 0 |
| pRT CF8 | 0 | 0 | - | 0 |
| pRT CF9 | 0 | 0 | - | 0 |
| pRT CF10 | 0 | 0 | - | 0 |
| pRT CF XS | 9 | 0,8 | 1,2 | 3,6 |
| pRT 35S | 100 | 100 | 100 | 100 |

Wie die in Tabelle 3 aufgeführten Ergebnisse zeigen, zeigen die in den Konstrukten pRT CF 2 - 5 enthaltenen CFDV-Fragmente eine deutlich höhere Promotoraktivität in Tabak-Protoplasten als der *Xho*I/*Sty*I-CFDV-Fragment-Promotor des Konstrukts pRT CF XS, der zusätzlich den Translationsstart des offenen Leserasters ORF1 enthält und in dem deutschen Patent P 43 06 832 beschrieben worden ist.

Die Konstrukte pRT CF 7 - 10 zeigen in Tabak-Protoplasten keinerlei Aktivität, was zeigt, daß die Möglichkeit der Ausbildung der "stem-loop"-Struktur im Bereich der Nukleotide 941 bis 971 in dem CFDV-Fragment-Promotor für die Promotoraktivität in Pflanzenzellen essenziell ist.

Das Konstrukt pRTCF4 zeigt in Tabak-Protoplasten darüberhinaus eine vergleichbare Promotoraktivität wie der CaMV 35S-Promotor (vgl. Konstrukt pRT 35S).

## Patentansprüche

1. Verwendung einer aus CFDV stammenden DNA als Promotor zur Expression von Genen in Bakterien und Pilzen, insbesondere Hefen, wobei die aus CFDV stammende DNA eine Länge von 1291 Nukleotiden und die in Fig. 1 dargestellte Struktur aufweist.

2. Verwendung von Fragmenten der 1291 Nukleotide umfassenden CFDV-DNA, die die in Fig. 1 dargestellte Struktur aufweist, als Promotor zur Expression von Genen in Bakterien und Pilzen.

3. Verwendung von Fragmenten der CFDV-DNA als Promotor nach Anspruch 2, **dadurch gekennzeichnet, daß** das CFDV-DNA-Fragment die wiederholte RPT-Struktur, die 52 bp-Sequenz und die TATAA-Sequenz umfaßt.

4. Verwendung von Fragmenten der CFDV-DNA als Promotor nach Anspruch 3, **dadurch gekennzeichnet, daß** das CFDV-DNA-Fragment die Nukleotide 211 bis 991, 409 bis 991, 611 bis 991, 711 bis 991, 211 bis 962, 409 bis 962, 611 bis 962 oder 711 bis 962 umfaßt oder das *Xho*I/*Sty*I-Restriktionsfragment der CFDV-DNA mit den Nukleotiden 1 bis 1157 ist, wobei für die Numerierung der Nukleotide dem 5'-Ende der aus der Spaltung der zirkulären CFDV-DNA mit der Restriktionsendonuklease *Xho*I resultierenden linearisierten DNA die Position 1 zugewiesen worden ist.

5. Verwendung von Fragmenten der CFDV-DNA als Promotor nach Anspruch 4, wobei das CFDV-Fragment die Nukleotide 611 bis 991 oder 611 bis 962 der CFDV-DNA umfaßt.

6. Verwendung von Derivaten der aus CFDV stammenden DNA oder von Derivaten der Fragmente der CFDV-DNA, wie sie in einem der Ansprüche 1 bis 5 definiert sind, als Promotor zur Expression von Genen in Bakterien und Pilzen, insbesondere Hefen, wobei die Derivate der aus CFDV stammenden DNA oder der CFDV-DNA-Fragmente durch Substitution, Deletion, Insertion oder Modifizierung von einzelnen Nukleotiden oder kleineren Gruppen von Nukleotiden von den Ausgangssequenzen abgeleitet sind und eine vergleichbare Promotoraktivität wie die Ausgangssequenz aufweisen.

7. Verwendung von aus CFDV stammender DNA oder von Fragmenten der CFDV-DNA oder von Derivaten davon, wie sie in einem der Ansprüche 1 bis 6 definiert sind, für die Herstellung von chimären Konstrukten zur transienten und stabilen Expression von Genen in Bakterien und Pilzen, insbesondere Hefen.

8. Bakterienzellen oder Pilzzellen, insbesondere Hefezellen, die mit aus CFDV stammender DNA oder Fragmenten der CFDV-DNA oder Derivaten davon, wie sie in einem der Ansprüche 1 bis 6 definiert sind, transformiert worden sind.

## Claims

1. Use of a CFDV-derived DNA as promoter for expressing genes in bacteria and fungi, in particular yeasts, where the CFDV-derived DNA has a length of 1291 nucleotides and the structure shown in Fig. 1.

2. Use of fragments of the CFDV DNA which encompasses 1291 nucleotides and has the structure shown in Fig. 1 as promoter for expressing genes in bacteria and fungi.

3. Use of CFDV DNA fragments as promoter according to Claim 2, **characterized in that** the CFDV DNA fragment encompasses the repeated RPT structure, the 52 bp sequence and the TATAA sequence.

4. Use of CFDV DNA fragments as promoter according to Claim 3, **characterized in that** the CFDV DNA fragment encompasses the nucleotides 211 to 991, 409 to 991, 611 to 991, 711 to 991, 211 to 962, 409 to 962, 611 to 962 or 711 to 962 or is the *Xho*I/*Sty*I restriction fragment of the CFDV DNA with the nucleotides 1 to 1157, where, for the purpose of numbering the nucleotides, the 5'-end of the linearized DNA resulting from cleaving the circular CFDV DNA with the restriction endonuclease *Xho*I has been assigned the position 1.

5. Use of CFDV DNA fragments as promoter according to Claim 4, where the CFDV fragment encompasses the nucleotides 611 to 991 or 611 to 962 of the CFDV DNA.

6. Use of derivatives of the CFDV-derived DNA or of derivatives of CFDV DNA fragments as they are defined in one of Claims 1 to 5 as promoters for expressing genes in bacteria and fungi, in particular yeasts, the derivatives of the CFDV-derived DNA or of the CFDV DNA fragments being derived from the starting sequences by substitution, deletion, insertion or modification of individual nucleotides or smaller groups of nucleotides and the promoter activity being similar to the starting sequence.

7. Use of CFDV-derived DNA or of CFDV DNA fragments or their derivatives as they are defined in one of Claims 1 to 6 for the preparation of chimeric constructs for the transient and stable expression of genes in bacteria and fungi, in particular yeasts.

8. Bacterial cells or fungal cells, in particular yeast cells, which were transformed with CFDV-derived DNA or CFDV DNA fragments or their derivatives as they are defined in one of Claims 1 to 6.

## Revendications

1. Utilisation d'un ADN provenant du CFDV comme promoteur de l'expression de gènes dans les bactéries et les champignons, notamment les levures, l'ADN provenant du CFDV ayant une longueur de 1291 nucléotides et la structure représentée à la figure 1.

2. Utilisation de fragments de l'ADN-CFDV comprenant 1291 nucléotides, qui présentent la structure représentée à la figure 1, comme promoteur de l'expression de gènes dans les bactéries et les champignons.

3. Utilisation de fragments d'ADN-CFDV comme promoteur selon la revendication 2, **caractérisée en ce que** le fragment d'ADN-CFDV comprend la structure RPT répétée, la séquence 52 bp et la séquence TATAA.

4. Utilisation de fragments d'ADN-CFDV comme promoteur selon la revendication 3, **caractérisée en ce que** le fragment d'ADN-CFDV comprend les nucléotides 211 à 991, 409 à 991, 611 à 991, 711 à 991, 211 à 962, 409 à 962, 611 à 962 ou 711 à 962 ou le fragment de restriction *Xho*I/*Sty*I et l'ADN-CFDV avec les nucléotides 1 à 1157, la position 1 étant attribuée à l'extrémité 5' de l'ADN linéarisé résultant de la fission de l'ADN-CFDV circulaire avec l'endonucléase de restriction *Xho*I pour la numérotation des nucléotides.

5. Utilisation de fragments de l'ADN-CFDV comme promoteur selon la revendication 4, le fragment CFDV comprenant les nucléotides 611 à 991 ou 611 à 962 de l'ADN-CFDV.

6. Utilisation de dérivés de l'ADN provenant du CFDV ou de dérivés des fragments d'ADN-CFDV, tels qu'ils sont définis dans l'une des revendications 1 à 5, comme promoteur de l'expression de gènes dans les bactéries et les champignons, notamment les levures, les dérivés de l'ADN provenant du CFDV ou des fragments de l'ADN-CFDV étant dérivés des séquences initiales par substitution, délétion, insertion ou modification de nucléotides individuels ou de petits groupes de nucléotides et présentant une activité promotrice comparable à celle de la séquence initiale.

7. Utilisation d'ADN provenant du CFDV ou de fragments d'ADN-CFDV ou de dérivés de ceux-ci, tels que définis dans l'une des revendications 1 à 6, pour la fabrication de constructions chimères pour l'expression transitoire et stable de gènes dans les bactéries et les champignons, notamment les levures.

8. Cellules bactériennes ou fongiques, notamment les cellules de levures, transformées avec de l'ADN provenant du CFDV ou des fragments d'ADN-CFDV ou des dérivés de ceux-ci, tels que définis dans l'une des revendications 1 à 6.
